# EUROPÄISCHE PATENTSCHRIFT

(11) **EP 0 425 929 B1**
(45) Veröffentlichungstag und Bekanntmachung des Hinweises auf die Patenterteilung: **06.12.1995**
(21) Anmeldenummer: 90120076.6
(22) Anmeldetag: 19.10.1990
(51) Int. Cl.: C07D 211/46, C07D 498/04

(54) **Verfahren zur Herstellung von Zwischenprodukten und zur Synthese von N-(2-Hydroxyethyl)-2-hydroxymethyl-3,4,5-trihydroxypiperidine**
Process for the preparation of N-(2-hydroxyethyl)-2-hydroxymethyl-3,4,5-trihydroxy-piperidine and of intermediates
Procédé pour la préparation de N-(2-hydroxyéthyl)-2-hydroxyméthyl-3,4,5-trihydroxy-pipéridine et d'intermédiaires

(30) Priorität: 01.11.1989 DE 3936295
(43) Veröffentlichungstag der Anmeldung: 08.05.1991
(73) Patentinhaber: BAYER AG, 51368 Leverkusen (DE)
(72) Erfinder: Hinsken, Werner, Dr., W-4300 Essen 14 (DE)

(56) Entgegenhaltungen:
- EP-A- 0 000 947
- EP-A- 0 019 899
- EP-A- 0 049 858
- EP-A- 0 055 431
- DE-A- 3 906 463
- SYNTHESIS, Nr. 10, Oktober 1987, Seiten 927-929; N. FARFAN et al.: "High-yield synthesis of N-(2-hydroxyethyl)-N-alkylglycine derivatives by reaction of ethanolamines with glyoxal"
- PATENT ABSTRACTS OF JAPAN, Band 3, Nr. 128 (C-62), 24. Oktober 1979; & JP-A-54 106 477

## Beschreibung

Die vorliegende Erfindung betrifft ein Verfahren zur Herstellung von N-(2-Hydroxyethyl)-2-hydroxymethyl-3,4,5-trihydroxypiperidine der allgemeinen Formel (I) 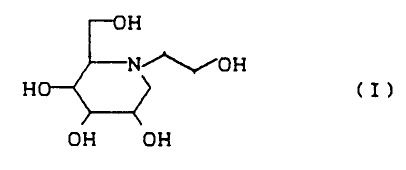
sowie Synthesezwischenprodukten der allgemeinen Formeln (II) und (III) 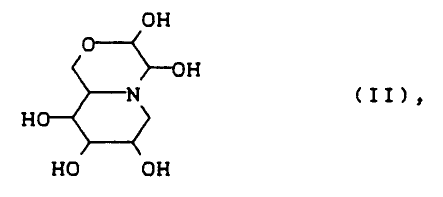 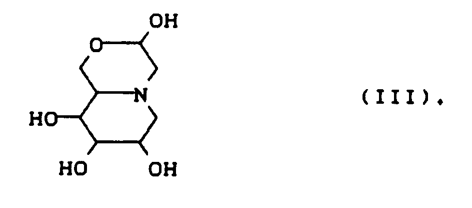
Verbindungen der Formel (I) sind sehr gute α-Glycosidasehemmer, insbesondere für Disaccharidasen. Zur Beeinflussung einer Vielzahl von Stoffwechselvorgängen stellen diese Verbindungen somit wertvolle Mittel dar.

Insbesondere kann die Verbindung der Formel (IV), die die bevorzugte stereoisomere Form der Verbindungen der Formel (I) beschreibt, als Mittel gegen Diabetes verwendet werden (EP 947 A1). 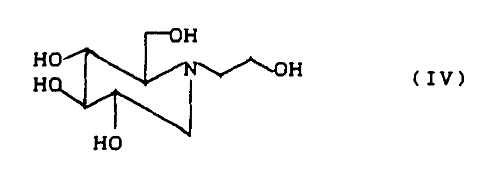
Die bekannten Wege zur Synthese der Verbindungen der Formel (I) gehen aus von 2-Hydroxymethyl-3,4,5-trihydroxypiperidinen (V) 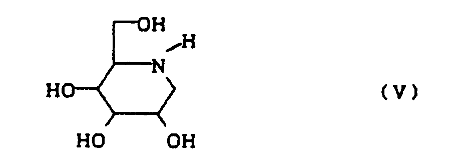
wobei die Verbindungen der Formel (V) mit Glykolaldehyd in Gegenwart eines Wasserstoff-Donor-Reduktionsmittels umgesetzt werden.

Ebenso ist bekannt, daß man Verbindungen der Formel (I) erhält, wenn man Verbindungen der Formel (V) mit Ethylenoxid in an sich bekannter Weise umsetzt (DE-OS 3 024 901 A1).

Alle weiteren bekannten Verfahren, die gleichfalls zur Herstellung von Verbindungen der Formel (I) führen, sind in der Europäischen Patentanmeldung 947 A1 und in den Deutschen Offenlegungsschriften 3 024 901 A1, 3 611 841 und 3 906 463 zitiert.

Die Verwendung gasförmiger cancerogener Einsatzstoffe, teure, schwer zugängliche Ausgangskomponenten sowie lange Reaktionszeiten seien beispielhaft genannt für die zahlreichen Nachteile der bisher beschriebenen Verfahren.

Durch das erfindungsgemäße Verfahren zur Herstelllung von Verbindungen der allgemeinen Formel (I) lassen sich diese Nachteile vermeiden.

Die Erfindung betrifft daher ein neues, chemisch eigenartiges Verfahren zur Herstellung von N-(2-Hydroxyethyl)-2-hydroxymethyl-3,4,5-trihydroxypiperidine der allgemeinen Formel (I) 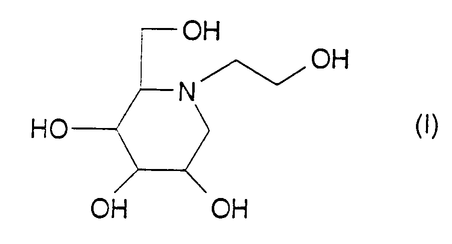
dadurch gekennzeichnet, daß Verbindungen der Formel (V)in Wasser oder einer Wasser/Alkohol-Mischung mit Glyoxal (VI) zum Additionsprodukt (II) umgesetzt werden, 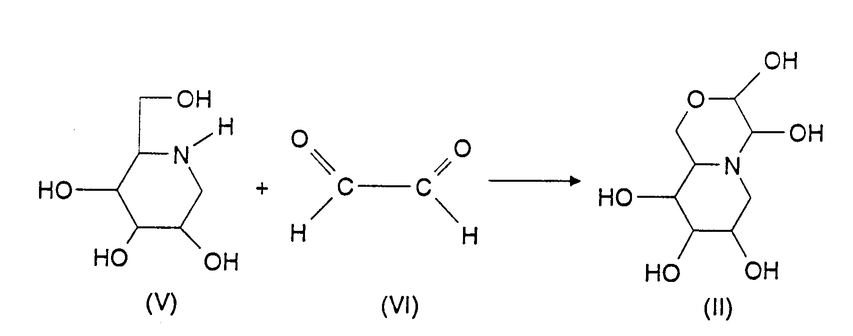
und danach unter reduktiven Bedingungen mit einem Wasserstoff-Donor-Reduktionsmittel bei pH-Werten zwischen 4,5 und 8,5 zum Zwischenprodukt (III) und anschließend durch Reaktion mit einem Wasserstoff-Donor-Reduktionsmittel bei pH-Werten zwischen 8,5 und 14 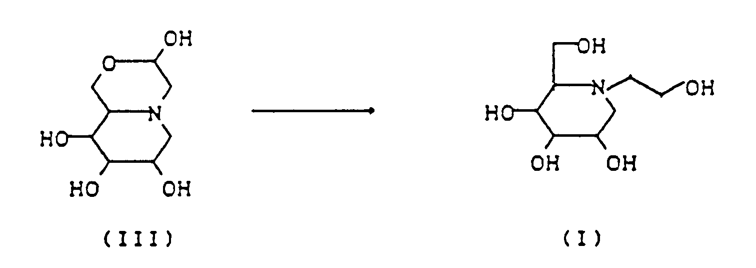
zu Verbindungen der Formel (I) reduziert werden.

Nach entsprechender Aufarbeitung erhält man die Verbindung (I) durch Kristallisation aus einem geeigneten Lösungsmittel oder Lösungsmittelgemisch.

Das Verfahren ist ebenso dadurch gekennzeichnet, daß man bei geeigneter Reaktionsführung und unter geeigneten Aufarbeitungsbedingungen die einzelnen Reaktionszwischenprodukte, das Additionsprodukt (II) sowie das Synthesezwischenprodukt (III) isolieren kann, welche ihrerseits wiederum als Ausgangsprodukte für weitere Synthesen dienen können.

Es ist bereits bekannt geworden, daß man bei der Umsetzung von N-Alkylaminoethanolen mit Glyoxal sowohl N-(2-Hydroxyethyl)-N-alkylglycine erhält [Synthesis 1987, 927] als auch Produktgemische bestehend aus N,N′-Dialkyl-3,3′-dioxazolidinen, N-Alkyl-2-oxomorpholinen, N-Alkyl-2,3-epoxymorpholinen sowie N-(2-Hydroxyethyl)-N-alkylglycine resultieren [Bull. Soc. Chim. Fr. 1978 II, 83]. Des weiteren ist bekannt geworden, daß cyclische sekundäre Amine mit Glyoxal unter Bildung von 1,1,2,2-Tetraaminoethanen reagieren. Ebenso ist die Bildung von Glycinamiden beschrieben [J. Heterocycl. Chem. 7, 1153 (1970)].

Es ist daher als ausgesprochen überraschend zu bezeichnen, daß gemäß der erfindungsgemäßen Umsetzung von 2-Hydroxymethyl-3,4,5-trihydroxypiperidinen (V) mit Glyoxal Reaktionsbedingungen gefunden wurden, die es erlauben, als hoch reines Reaktionsprodukt das Additionsprodukt der Formel (II) zu isolieren, da man im Hinblick auf den Stand der Technik erwarten mußte, daß sich neben N-Alkyl-glycinen und Tetraaminoethan-Derivaten auch Dioxazolidin-Verbindungen bilden sollten. Des weiteren wurde gefunden, daß bei der Umsetzung von Verbindungen der Formel (II) mit einem Wasserstoff-Donor-Reduktionsmittel in hohen Ausbeuten nach entsprechender Aufarbeitung Synthesezwischenprodukte der Formel (III) erhalten werden. Gleichfalls wurden Reaktionsbedingungen gefunden, Zwischenprodukte der Formel (III) durch Umsetzung mit einem Wasserstoff-Donor-Reduktionsmittel in hohen Ausbeuten und Reinheiten in Endprodukte der Formel (I) zu überführen, welche nach geeigneter Aufarbeitung kristallin erhalten werden.

Als eine Besonderheit der erfindungsgemäßen Umsetzung ist hervorzuheben, daß N-(2-Hydroxyethyl)-2-hydroxymethyl-3,4,5-trihydroxypiperidine (I) in hohen Ausbeuten und Reinheiten in einem Eintopf-Verfahren zugänglich sind, wenn Verbindungen der Formel (V) mit Glyoxal bei Temperaturen zwischen 15° bis 35°C zur Reaktion gebracht, anschließend mit einem Wasserstoff-Donor-Reduktionsmittel bei einem pH-Wert zwischen pH 4,5 bis pH 8,5 und Temperaturen zwischen 5° bis 80°C reduziert, auf einen pH-Wert zwischen pH 8,5 bis pH 14 eingestellt und erneut bei Temperaturen zwischen 30° bis 100°C mit einem Wasserstoff-Donor-Reduktionsmittel umgesetzt werden.

Dieses neuartige Verfahren zur N-Hydroxyethylierung von 2-Hydroxymethyl-3,4,5-trihydroxypiperidinen (V) weist somit eine Reihe von Vorteilen auf. Neben dem leicht zugänglichen, preiswerten Einsatzstoff, der zur Anwendung kommt, wird der Einsatz von hochreaktiven, schwer handhabbaren, gasförmigen sowie canzerogenen Einsatzstoffen vermieden. N-(2-Hydroxyethyl)-2-hydroxymethyl-3,4,5-trihydroxypiperidine (I) lassen sich somit wesentlich preiswertiger und sicherer herstellen.

Verwendet man bei dem erfindungsgemäßen Verfahren als Edukt beispielsweise Desoxymannonojirimycin und Glyoxal (30 %ige Lösung) mit Wasser als Verdünnungsmittel, so läßt sich der Ablauf der Reaktion durch folgendes Formelschema beschreiben. 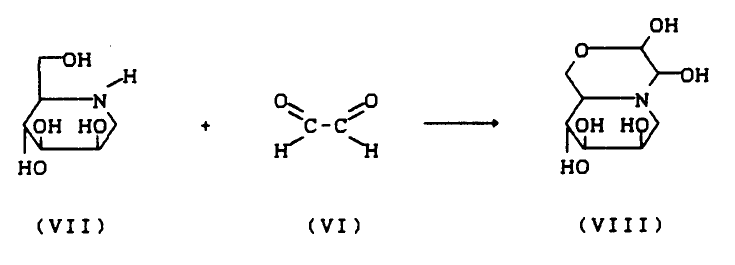
Versetzt man das Additionsprodukt der Formel (VIII) mit Natriumborhydrid als Wasserstoff-Donor-Reduktionsmittel im wäßrigen Milieu unter pH-Kontrolle, so läßt sich das folgende Formelschema formulieren. 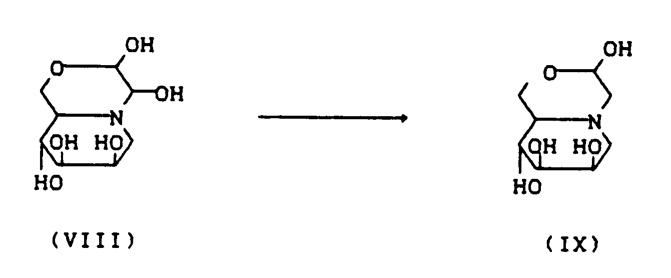
Wird das Reduktionsprodukt (IX) weiter mit Natriumborhydrid im basisch-wäßrigen Milieu umgesetzt, so gibt das unten stehende Formelschema den Reaktionsverlauf wieder. 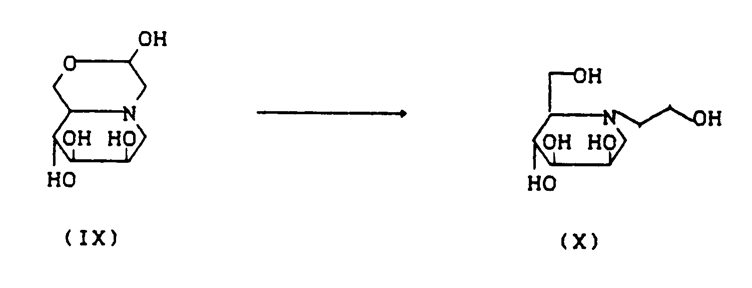
Durch Aufziehen auf einen sauren Ionenaustauscher und Elution mit wäßriger Ammoniak-Lösung erhält man nach Konzentrierung der produkthaltigen Lösungen und Kristallisation aus Alkohol/Wasser das Reaktionsprodukt 1,5-Didesoxy-1,5-[(2-hydroxyethyl)-imino]-D-mannitol (X).

Für den Reaktionsverlauf ist es unerheblich, ob die chemischen Einzelschritte getrennt, nach entsprechender Isolierung der Reaktionsprodukt, durchgeführt werden oder das Zielmolekül erst nach der Reaktionssequenz gemäß einem Eintopfverfahren isoliert wird.

Die bevorzugte Variante stellt die Eintopf-Reaktion dar, da aufarbeitungsbedingte Ausbeuteverluste entfallen.

Die als Ausgangsstoffe eingesetzten Aminozucker der allgemeinen Formel (V) sind nach bekannten Methoden zugänglich [DE 3 611 841 A1, Angew. Chem. 100, 737 (1988), Carbohydr. Res. 167, 305 (1987)].

Das als weiterer Einsatzstoff dienende Glyoxal kann in allen handelsüblichen Formen zur Anwendung kommen. Bevorzugt werden wäßrige Lösungen sowie festes Glyoxal (trimer). Besonders bevorzugt werden 30 %ige und 40 %ige wäßrige Lösungen.

Die bevorzugte Herstellung des Additionsproduktes der allgemeinen Formel (II) kann durch die Wahl der Reaktionsbedingungen, insbesondere der Temperatur, beeinflußt werden. Es hat sich gezeigt, daß zur Erzielung einer hohen Ausbeute eines hoch reinen Reaktionsproduktes (II) die Reaktionstemperatur so zu wählen ist, daß ein Intervall von +5°C bis +50°C, bevorzugt ein Intervall von +15°C bis +35°C eingehalten wird. Dazu kann so vorgegangen werden, daß zu einer Lösung aus 2-Hydroxymethyl-3,4,5-trihydroxypiperidin der allgemeinen Formel (V) in Wasser oder einem Wasser-Alkohol-Gemisch, wobei als Alkohole solche mit 1 bis 4 Kohlenstoffatomen, vorzugsweise Methanol oder Ethanol, oder Etheralkohole wie Methylglykol oder Ethylglykol zur Anwendung kommen, Glyoxal als Lösung oder Feststoff, gegebenenfalls verdünnt mit Wasser oder als Suspension, bevorzugt 30 %ige oder 40 %ige wäßrige Lösungen, gegebenenfalls unter Kühlung, zugegeben wird. Die Reaktionstemperatur soll dabei den oben angegebenen Werten entsprechen.

Bei der Durchführung ist das Verhältnis der an der Reaktion beteiligten Stoffe breit variierbar. Im allgemeinen werden zur Herstellung von 1 Mol der Verbindung der Formel (II) 1 Mol der Verbindung der Formel (V) mit 1 Mol bis 3 Mol Glyoxal umgesetzt, bevorzugt 1 Mol bis 2 Mol, besonders bevorzugt 1 Mol bis 1,5 Mol Glyoxal. Durch Gefriertrocknung oder andere, dem Stand der Technik entsprechende Methoden zur Entfernung von Lösungsmitteln, können Verbindungen der allgemeinen Formel (II) hoch rein erhalten werden.

Die Additionsverbindungen (II) lassen sich einfach durch Reaktion mit einem Wasserstoff-Donor-Reduktionsmittel in Verbindungen der allgemeinen Formel (III) überführen. Die Reduktion kann hierbei zum einen durch katalytische Hydrierung an geeigneten Katalysatoren erfolgen, wobei die Additionsverbindung (II) in Wasser oder einem Wasser-Alkohol-Gemisch gelöst vorgelegt wird, oder es wird die aus der obig beschriebenen Verfahrensweise direkt erhaltene Lösung eingesetzt. Die Hydrierung erfolgt an Metall- oder Edelmetall-Katalysatoren, wobei die Katalysatorkonzentration zwischen 0,5 Mol-% bis 20 Mol-% liegt, bevorzugt 0,5 Mol-% bis 10 Mol-%. Der Wasserstoffdruck und die Reaktionstemperatur können bei der katalytischen Hydrierung in einem großen Bereich variiert werden. So können Reaktionstemperaturen zwischen 10°C und 100°C und Wasserstoffdrücke zwischen 10 bar und 200 bar gewählt werden. Bevorzugt werden Temperaturen zwischen 40°C und 70°C und Wasserstoffdrücke zwischen 30 bar und 80 bar.

Zum anderen kann die Reaktion mit komplexen Borhydriden erfolgen, die auch die bevorzugte Verfahrensvariante darstellt.

Bei dieser Verfahrensvariante können die gleichen wäßrigen oder wäßrig-alkoholischen Lösungen der Additionsverbindung (II) eingesetzt werden wie bei der vorhergehenden Variante beschrieben. Zur Erzielung hoher Ausbeuten und besonders reiner Verbindungen ist es notwendig, im Falle der Anwendung von komplexen Borhydriden die Umsetzung nur in einem recht engen pH-Wert-Bereich von pH 4,5 bis pH 8,5, vorteilhaft pH 5,5 bis pH 7,5, durchzuführen. Die wäßrige oder wäßrig-alkoholische Lösung der Additionsverbindung (II) wird hierzu mit einer Lösung oder Suspension des entsprechenden komplexen Borhydrids versetzt, wobei durch synchrone Zugabe einer Säure die Einstellung des gewünschten pH-Wertes, gegebenenfalls unter Kühlung, erfolgt und die pH-Wert-Messung z.B. mit einer Glaselektrode durchgeführt werden kann. Als Säuren kommen z.B. Mineralsäuren in Frage, vorzugsweise Salzsäure und Schwefelsäure. Die Säure wird vorzugsweise als verdünnte wäßrige oder wäßrig-alkoholische Lösung eingesetzt, wobei Konzentrationen von 5 bis 35 Gew.-% im Falle der Salzsäure und 5 bis 90 Gew.-% im Falle der Schwefelsäure möglich sind.

Bei den komplexen Borhydriden kommen bevorzugt Dialkylaminoborane, Alkaliboranate und Natriumcyanoborhydrid zur Anwendung. Ganz besonders bevorzugt sind Natriumboranat NaBH₄ und Dimethylaminoboran BH₃N(CH₃)₂. Ihre Zugabe kann portionsweise oder kontinuierlich als Suspension oder vorteilhaft in Form einer wäßrigen oder wäßrig-alkoholischen Lösung erfolgen. Das Molverhältnis von Additionsprodukt (II) zum Reduktionsmittel sollte etwa 1 zu 0,4 bis 1,5, vorzugsweise 1 zu 0,6 bis 1 zu 1,0 sein. Die Reduktion kann bei Temperaturen von 10° bis 80°C erfolgen, bevorzugt werden Temperaturen zwischen 20° und 40°C.

Nach beendeter Reaktion wird der Überschuß an Reduktionsmittel durch Zugabe von Säure oder eines reaktiven Ketons zerstört.

Zur Isolierung und Reinigung des Reaktionsproduktes der allgemeinen Formel (III) kann je nach Verfahrensvariante direkt aus der gegebenenfalls konzentrierten Ansatzlösung kristallisiert werden oder es wird an geeigneten Ionenaustauschern oder Kieselgel chromatographiert oder aber man zieht das Produkt auf einen sauren Ionenaustauscher auf, eluiert mit gegebenenfalls verdünnter Ammoniak- oder Aminlösung, konzentriert die produkthaltige Fraktion und kristallisiert aus geeigneten Lösungsmitteln um.

Gleich den Verbindungen der Formel (III) sind die Zielmoleküle der Formel (I) durch Reduktion mit einem Wasserstoff-Donor-Reduktionsmittel zugänglich. Hierzu können wäßrige oder wäßrig-alkoholische Lösungen der Verbindungen (III) sowie die aus der obigen beschriebenen Reduktion erhaltenen Lösungen eingesetzt werden.

Zur Erzielung einer hinreichenden Reaktionsgeschwindigkeit ist die Wahl des pH-Wertes bei der Umsetzung wesentlich. Sowohl bei der katalytischen Hydrierung als auch bei der Reduktion mit komplexen Borhydriden ist ein pH-Bereich von pH 8,5 bis pH 14, vorteilhaft pH 9 bis pH 11 zu wählen.

Als Basen zur pH-Einstellung können alle Hydroxide und Carbonate verwendet werden, soweit sie ausreichend in Wasser sowie in wäßrig-alkoholischen Mischungen löslich sind. Sie können sowohl in fester Form als auch in Lösung eingesetzt werden, wobei die zu wählende Einsatzkonzentration beliebig ist.

Bei der katalytischen Hydrierung erfolgt die Reaktion nach der pH-Einstellung unter Zusatz von Metall- oder Edelmetall-Katalysatoren mit einer Katalysatorkonzentration zwischen 0,1 Mol-% bis 20 Mol-%, bevorzugt 0,4 Mol-% bis 10 Mol-%.

Die Reaktionstemperatur liegt hierbei zwischen 20°C bis 150°C, bevorzugt 50°C bis 90°C bei einem Wasserstoffdruck zwischen 10 bar und 250 bar, bevorzugt 40 bar bis 80 bar.

Die erhaltene oder hergestellte wäßrige oder wäßrig-alkoholische Lösung der Verbindungen (III) kann ebenso mit komplexen Borhydriden in die Zielmoleküle (I) überführt werden, welches die bevorzugte Verfahrensvariante darstellt.

Hierzu wird die wäßrige oder wäßrig-alkoholische Lösung der Verbindung (III) nach der Einstellung des pH-Wertes mit einer Lösung oder Suspension des entsprechenden komplexen Borhydrides versetzt, wobei die Zugabe portionsweise oder kontinuierlich als Suspension oder vorteilhaft in Form einer wäßrigen oder wäßrig-alkoholischen Lösung, gegebenenfalls unter Kühlung, erfolgen kann. Das Molverhältnis von Verbindungen der Formel (III) zum Reduktionsmittel sollte etwa 1 zu 0,4 bis 1,5, vorzugsweise 1 zu 0,6 bis 1 zu 1,0 sein. Die Reduktion kann bei Temperaturen von 20°C bis 90°C erfolgen, bevorzugt werden Temperaturen zwischen 40°C und 70°C. Bei den komplexen Borhydriden kommen bevorzugt Alkaliboranate und Dialkylaminoborane zur Anwendung. Ganz besonders bevorzugt ist Natriumboranat NaBH₄.

Nach beendeter Reaktion wird der Überschuß an Reduktionsmittel durch Zugabe von Säure oder eines reaktiven Ketons zerstört.

Zur Isolierung und Reinigung des Zielmoleküls (I) wird an geeigneten Ionenaustauschern oder Kieselgel chromatographiert oder aber man zieht das Produkt auf einen sauren Ionenaustauscher auf, eluiert mit gegebenenfalls verdünnter Ammoniak- oder Aminlösung, konzentriert die produkthaltigen Fraktionen auf und kristallisiert aus geeigneten Lösungsmitteln um.

Als saure Ionenaustauscher können prinzipiell alle schwach und stark sauren Typen zur Anwendung kommen. Sie können sowohl gelförmig als auch makroporös sein.

### Beispiel 1

### (7S, 8R, 9R, 9aR)-3,4,7,8,9-Pentahydroxy-octahydro-pyrido[2,1-c][1,4]oxazin

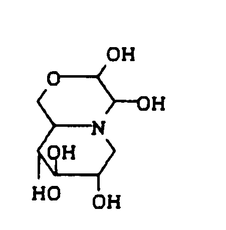

Eine Lösung aus 10 g 1,5-Didesoxy-1,5-amino-D-glucitol in 20 ml entsalztem Wasser werden bei Raumtemperatur mit 7,7 ml 40 %ige wäßrige Glyoxallösung versetzt und bei dieser Temperatur 30 Minuten gerührt. Die Lösung wird eingefroren und das Lösungsmittel mittels Gefriertrocknung entfernt. Die Kristalle werden gemörsert in wenig Diisopropylether angeschlämmt und ca. 30 Minuten bei Raumtemperatur gerührt. Das Produkt wird durch Filtration isoliert.
Ausbeute: 13 g (95,8 % der Theorie)

### Massenspektrum:

Der wichtigste Peak im oberen Massenbereich liegt bei m/e = 203 (M-H₂O).

Die Substanz ist ein Gemisch diasteromerer Verbindungen.

### Beispiel 2

### (7S, 8R, 9S, 9aR)-3,4,7,8,9-Pentahydroxy-octahydro-pyrido[2,1-c][1,4]oxazin

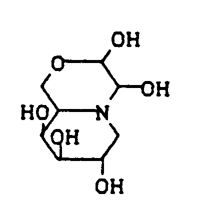

Die Herstellung erfolgt analog Beispiel 1 aus 1,5-Didesoxy-1,5-imino-D-galactitol und entsprechenden Molverhältnissen an 30 %igem wäßrigen Glyoxal.
Ausbeute: 12,8 g (94 % der Theorie)

| C₈H₁₅NO₆ (221,2) | | | |
|---|---|---|---|
| Ber.: | C 43,4 % | H 6,8 % | N 6,3 % |
| Gef.: | C 43,2 % | H 6,6 % | N 6,5 % |

### Beispiel 3

### (7S, 8R, 9S, 9aR)-3,7,8,9-Tetrahydroxy-octahydro-pyrido[2,1-c][1,4]oxazin

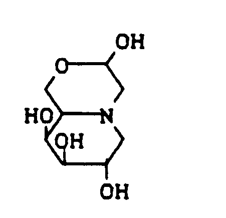

### Methode A:

50 g der Verbindung aus Beispiel 2 werden in 400 ml entsalztem Wasser gelöst und mit 4 g Pd/C-10 %ig versetzt. Es wird bei 60°C und 50 bar Wasserstoffdruck hydriert, wobei der Reaktionsverlauf mittels HPLC verfolgt wird. Es wird vom Kontakt abgesaugt, die Lösung konzentriert und aus Isopropanol/entsalztem Wasser umkristallisiert.
Ausbeute: 34,8 g (75,2 % der Theorie)

### Methode B:

50 g der Verbindung aus Beispiel 2 werden in 350 ml entsalztem Wasser gelöst. Mit 15 %iger Schwefelsäure wird ein pH-Wert von pH 5 bis pH 5,5 eingestellt. Man versetzt das Reaktionsgemisch anschließend kontinuierlich mit einer Lösung aus 7,0 g Natriumborhydrid in 30 ml entsalztem Wasser. Der pH-Wert wird durch Zutropfen einer 15 %igen Schwefelsäure zwischen pH 6,5 bis pH 7,1, die Temperatur durch Kühlen bei 20 bis 35°C gehalten. Es wird 30 Minuten nachgerührt und die Rohlösung anschließend über einen sauren Ionenaustauscher in der H^{⊕}-Form gespült. Der Austauscher wird mit entsalztem Wasser gewaschen und anschließend mit 5 %iger Ammoniaklösung eluiert. Die Produktfraktionen werden konzentriert und der Rückstand aus Isopropanol/entsalztem Wasser umkristallisiert.
Ausbeute: 39,5 g (85,3 % der Theorie)

### Massenspektrum:

Die wichtigsten Peaks im oberen Massenbereich liegen bei m/e = 205 und m/e = 188. Weitere Peaks findet man bei m/e = 146, m/e = 55.

Die Substanz ist ein Gemisch zweier diastereomerer Verbindungen.

Unter Beachtung der molaren Verhältnisse können direkt die im Beispiel 2 nach der Nachrührzeit erhaltenen Lösungen zur Umsetzung nach Methode A oder Methode B eingesetzt werden.

### Beispiel 4

### (7S, 8R, 9R, 9aR)-3,7,8,9-Tetrahydroxy-octahydro-pyrido[2,1-c][1,4]oxazin

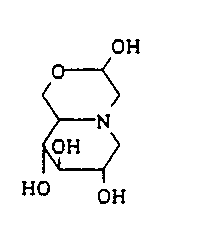

Die Darstellung erfolgt analog Beispiel 3 aus 50 g der Verbindung aus Beispiel 1 und den jeweils bei den verschiedenen Methoden angegebenen Einsatzstoffen.
Ausbeute:

| | |
|---|---|
| Methode A: | 35,4 g (76,4 % der Theorie) |
| Methode B: | 39,3 g (84,6 % der Theorie) |

| C₈H₁₅NO₅ (205,2) | | | |
|---|---|---|---|
| Ber.: | C 46,8 % | H 7,4 % | N 6,8 % |
| Gef.: | C 46,6 % | H 7,6 % | N 6,9 % |

Die Substanz ist ein Gemisch zweier diastereomerer Verbindungen.

Unter Beachtung der molaren Verhältnisse können direkt die in Beispiel 1 nach der Nachrührzeit erhaltenen Lösungen zur Umsetzung nach Methode A oder Methode B eingesetzt werden.

### Beispiel 5

### Herstellung von 1,5-Didesoxy-1,5-[(2-hydroxyethyl)-imino]-D-glucitol

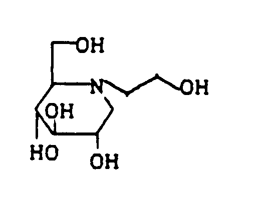

### Methode A:

100 g der Verbindung aus Beispiel 4 werden in 600 ml entsalztem Wasser gelöst und mit 45 %iger Natronlauge wird ein pH-Wert von pH 9 eingestellt. Bei Raumtemperatur tropft man eine Lösung aus 12 g Natriumboranat in 50 ml entsalztem Wasser hinzu. Man läßt die Temperatur auf 50°C kommen und halt diese Temperatur. Der Reaktionsverlauf wird mittels HPLC verfolgt. Nach beendeter Reaktion stellt man mit 15 %iger Schwefelsäure auf pH 7 ein und gibt die Rohlösung über einen sauren Ionenaustauscher in der H^{⊕}-Form. Der Austauscher wird mit entsalztem Wasser gewaschen und anschließend mit 6 %iger Ammoniaklösung eluiert. Die Produktfraktionen werden konzentriert und der Rückstand aus Ethanol/entsalztem Wasser umkristallisiert.
Ausbeute: 84,3 g (83,6 % der Theorie)

### Methode B (Eintopfverfahren):

100 g 1,5-Didesoxy-1,5-imino-D-glucitol werden in 200 ml entsalztem Wasser gelöst und mit 91 ml Glyoxal (40 %ig) bei Raumtemperatur versetzt. Man rührt 30 Minuten nach, stellt mit 10 %iger Salzsäure pH 5 bis pH 5,5 ein und versetzt mit einer Lösung aus 14 g Natriumboranat in 50 ml entsalztem Wasser. Der pH-Wert wird durch Zutropfen einer 10 %igen Salzsäure zwischen pH 6,5 bis pH 7,1, die Temperatur durch Kühlen zwischen 20° bis 40°C gehalten. 30 Minuten nach beendeter Zugabe wird bei 20°C mit 30 %iger Natronlauge pH 9 eingestellt und anschließend eine Lösung aus 17,4 g Natriumboranat in 80 ml entsalztem Wasser hinzugefügt. Man läßt die Temperatur auf 50°C kommen und hält diese Temperatur. Der Reaktionsverlauf wird mittels HPLC verfolgt. Nach beendeter Reaktion wird mit 10 %iger Salzsäure auf pH 7 eingestellt und die Rohlösung über einen sauren Ionenaustauscher in der H^{⊕}-Form gegeben. Der Austauscher wird mit entsalztem Wasser gewaschen und anschließend mit 6 %iger Ammoniaklösung eluiert. Die Produktfraktionen werden konzentriert und der Rückstand aus Ethanol/entsalztem Wasser umkristallisiert.
Ausbeute: 109,7 g (86,3 % der Theorie)
Fp.: 145-147°C

### Beispiel 6

### Herstellung von 1,5-Didesoxy-1,5-[(2-hydroxyethyl)-imino]-D-galactitol

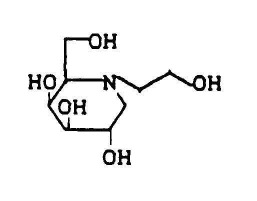

Die Darstellung erfolgt analog Beispiel 5 gemäß
Methode A aus 100 g der Verbindung aus Beispiel 3 und den bei der Methode A angegebenen Einsatzstoffen.
Ausbeute: 81,1 g (80,3 % der Theorie)
oder gemäß
Methode B aus 100 g 1,5-Didesoxy-1,5-imino-D-galactitol und den bei der Methode B angegebenen Einsatzstoffen.
Ausbeute: 104,8 g (82,5 % der Theorie)

### Massenspektrum:

Der wichtigste Peak im oberen Massenbereich liegt bei m/e = 176 (M - CH₂OH).

## Patentansprüche

1. Verfahren zur Herstellung von N-(2-Hydroxyethyl)-2-hydroxymethyl-3,4,5-trihydroxypiperidine der allgemeinen Formel (I) 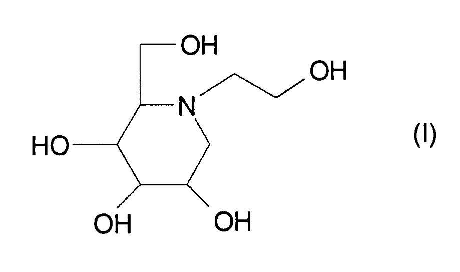 dadurch gekennzeichnet, daß Verbindungen der Formel (V)in Wasser oder einer Wasser/Alkohol-Mischung mit Glyoxal (VI) zum Additionsprodukt (II) umgesetzt werden, 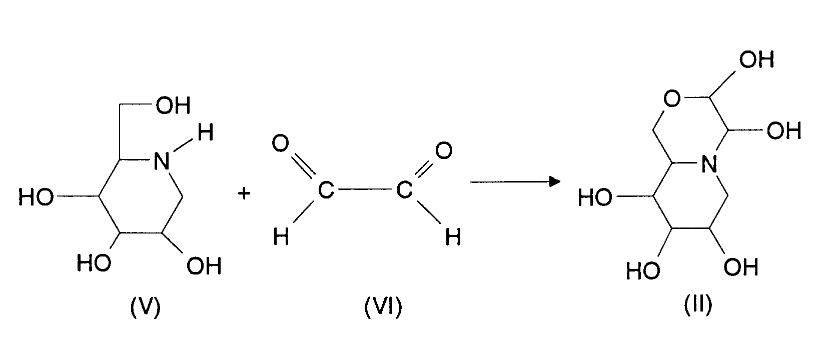 und danach unter reduktiven Bedingungen mit einem Wasserstoff-Donor-Reduktionsmittel bei pH-Werten zwischen 4,5 und 8,5 zum Zwischenprodukt (III) und anschließend durch Reaktion mit einem Wasserstoff-Donor-Reduktionsmittel bei pH-Werten zwischen 8,5 und 14 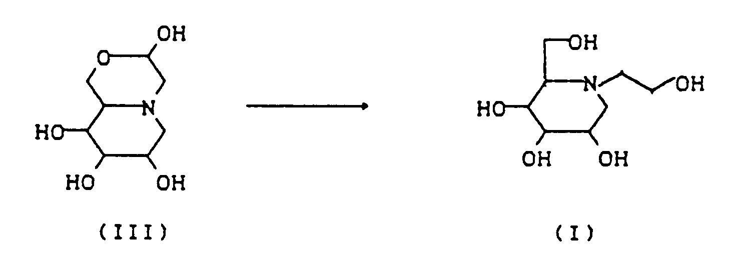 zu Verbindungen der Formel (I) reduziert werden.

2. Verfahren gemäß Anspruch 1 zur Herstellung von Verbindungen aus der Gruppe
a) (7S, 8R, 9R, 9aR)-3,4,7,8,9-Pentahydroxy-octahydro-pyrido[2,1-c][1,4]oxazin,
b) (7S, 8R, 9S, 9aR)-3,4,7,8,9-Pentahydroxy-octahydro-pyrido[2,1-c][1,4]oxazin,
c) (7S, 8R, 9S, 9aR)-3,7,8,9-Tetrahydroxy-octahydro-pyrido[2,1-c][1,d]oxazin und
d) (7S, 8R, 9H, 9aR)-3,7,8,9-Tetrahydroxy-octahydro-pyrido[2,1-c][1,4]oxazin.

## Claims

1. Process for the preparation of N-(2-hydroxyethyl)-2-hydroxymethyl-3,4,5-trihydroxypiperidines of the general formula (I) 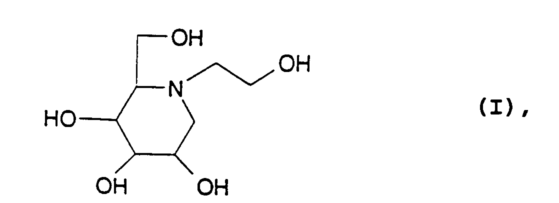 characterized in that compounds of the formula (V) are reacted in water or a water/alcohol mixture with glyoxal (VI) to give the addition product (II) 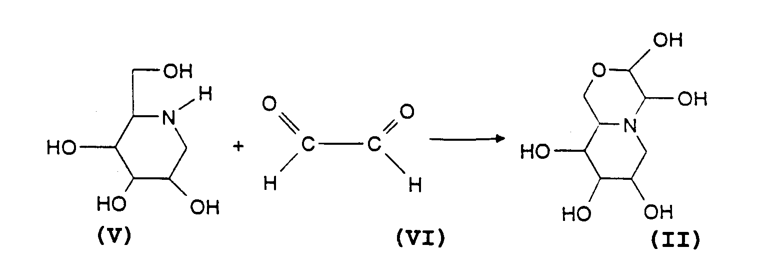 and this is then reduced under reductive conditions with a hydrogen donor reducing agent at pH values between 4.5 and 8.5 to give the intermediate (III) and subsequently by reaction with a hydrogen donor reducing agent at pH values between 8.5 and 14 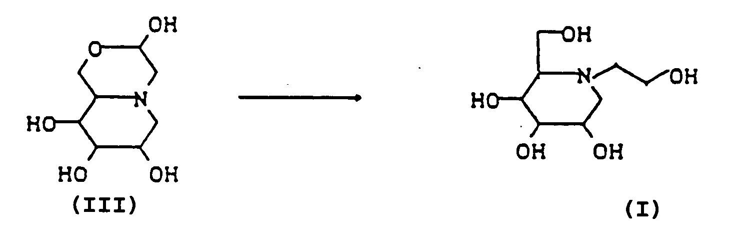 to give compounds of the formula (I).

2. Process according to Claim 1 for the preparation of compounds from the group
a) (7S, 8R, 9R, 9aR)-3,4,7,8,9-pentahydroxy-octahydro-pyrido[2,1-c][1,4]oxazine,
b) (7S, 8R, 9S, 9aR)-3,4,7,8,9-pentahydroxy-octahydro-pyrido[2,1-c][1,4]oxazine,
c) (7S, 8R, 9S, 9aR)-3,7,8,9-tetrahydroxy-octahydro-pyrido[2,1-c][1,4]oxazine and
d) (7S, 8R, 9R, 9aR)-3,7,8,9-tetrahydroxy-octahydro-pyrido[2,1-c][1,4]oxazine.

## Revendications

1. Procédé pour la préparation de N-(2-hydroxyéthyl)-2-hydroxyméthyl-3,4,5-trihydroxypipéridines répondant à la formule générale (I) 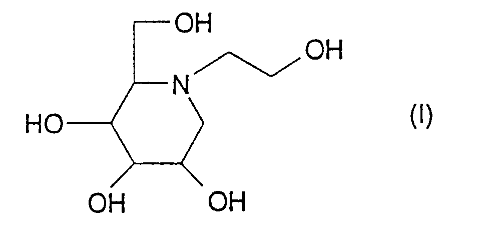 caractérisé en ce qu'on fait réagir des composés de formule (V) dans de l'eau ou dans un mélange eau/alcool avec du glyoxal (VI) pour obtenir le produit d'addition (II) 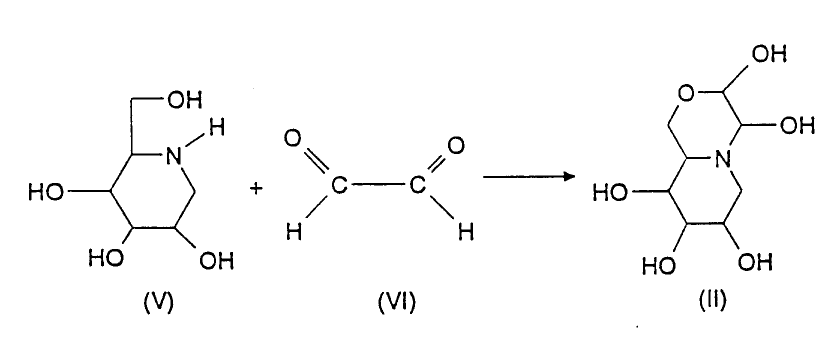 que l'on soumet ensuite à une réduction dans des conditions de réduction, avec un agent de réduction donneur d'hydrogène à des valeurs de pH entre 4,5 et 8,5 pour obtenir le produit intermédiaire (III) que l'on met ultérieurement à réagir avec un agent de réduction donneur d'hydrogène, à des valeurs de pH entre 8,5 et 14, 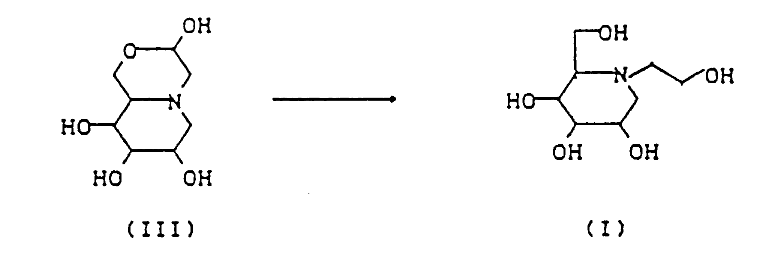 pour obtenir les composés de formule (I).

2. Procédé selon la revendication 1, pour la préparation de composés choisis parmi le groupe comprenant
a) la (7S, 8R, 9R, 9aR)-3,4,7,8,9-pentahydroxy-octahydro-pyrido[2,1-c][1,4]oxazine,
b) la (7S, 8R, 9S, 9aR)-3,4,7,8,9-pentahydroxy-octahydro-pyrido[2,1-c][1,4]oxazine,
c) la (7S, 8R, 9S, 9aR)-3,7,8,9-tétrahydroxy-octahydro-pyrido[2,1-c][1,4]oxazine, et
d) la (7S, 8R, 9R, 9aR)-3,7,8,9-tétrahydroxy-octahydro-pyrido[2,1-c][1,4]oxazine.
